# EUROPEAN PATENT APPLICATION

(11) **EP 3 103 419 A1**
(43) Date of publication of application: **14.12.2016**
(21) Application number: 15746193.0
(22) Date of filing: 02.02.2015
(51) Int. Cl.: A61F 5/44, A61F 5/451

(54) **BEDPAN DIAPER**

(30) Priority: 04.02.2014 JP 2014019757
(71) Applicant: Saitoh, Keiko, Miyagi 980-0013 (JP)
(72) Inventor: Saitoh, Keiko, Miyagi 980-0013 (JP)
(74) Representative: Gill Jennings & Every LLP
(86) International application number: PCT/JP2015/000449
(87) International publication number: WO 2015/118851

(57) **Abstract**

**[Summary]**

**[Problems to be solved]** To provide a diaper for insert-type toilet bowl which enables to set the insert-type toilet bowl and the diaper for insert-type toilet bowl efficiently in a short time and to comfortably be worn for a long time.

**[Means for solving the problems]** The excretion hole 74 is formed at the diaper main body 67 corresponding to said defecated fecal receiving portion 14 in the insert-type toilet bowl 9 comprising the defecated fecal receiving portion 14 and the hip receiving portion 13, and in the diaper for insert-type toilet bowl 66 used by setting inside the insert type toilet bowl 9, the toilet seat receiving sheet 78 for configuring the plug-in hole 79 of the hip receiving portion 13 is laid on at the outer surface of said diaper main body 67, the depth of the plug-in hole 79 is configured in such a manner that when the hip receiving portion 13 is inserted, the defecated fecal receiving portion 14 and the excretion hole 74 get together; and the toilet seat receiving sheet 78 has hygroscopic and waterproof properties and dimensions by which al least the defecated fecal receiving portion 14 can be wrapped up from the downside of the insert-type toilet bowl 9.

## Description

### Technical field

The present invention relates to a diaper for insert-type toilet bowl for persons who require nursing cares. More particularly, the invention relates to a diaper for inert-type toilet bowl which an immobile person requiring a high-level nursing cares uses with an insert-type toilet bowl placing between the legs to automatically process the urination / defecation by excretion processing device, and which enables to prevent leakage of urine or odor as far as possible and keep the region between the legs cleanly and reduce unpleasant sensation.

### Background art

The inventors et al. of the patent application have already proposed a diaper for insert-type toilet bowl used with an insert-type toilet bowl which a person requiring nursing care wears at a region between the legs in order to automatically detect a urination / defection by cared person such as the elderly confined to bed and process the excretion. (Patent literatures 1, 2, and 3)

The product shown in the patent literature 1 is one which is improved from the product written in the patent literature 2, as shown in Fig.9. The product shown in the patent literature 3 is almost the same as that written in the patent literature 1.

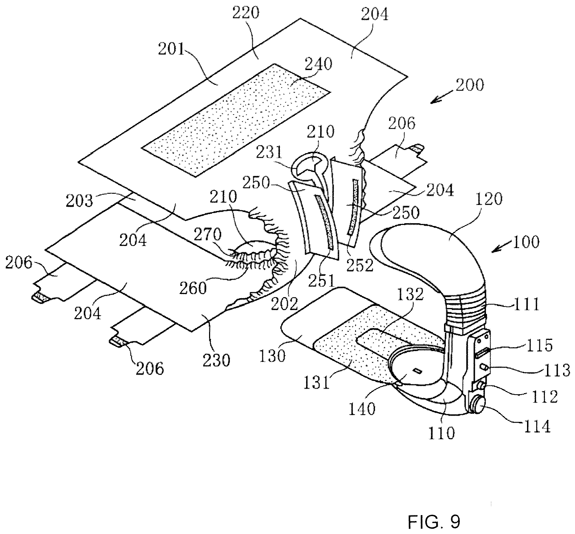

In Fig.9, individual portions are indicated as follows: 100: Excretion helping tool, 110: Main body, 111: Movable connecting portion, 112: Rinse-solution supply port, 113: Warm-air supply port, 114: Excretion port, 115: Connecting terminal, 120: Anterior cup portion, 130: Gluteal supporting portion, 131: Cushion layer, 132: Hollow portion, 140: Opening portion, 200: Cover for wearing the excretion helping tool, 201: Front body region, 202: Inside leg region, 203: Back body region, 204: Side flap, 206: Double-fastener tub, 210: Opening portion, 220: Top sheet, 230: Back sheet, 231: Peel-off paper, 240: Face fastener 250: Retentive sheet, 251: Face fastener, 252: Face fastener, 260: Three-dimensional gather, 270: Leakage preventive barrier.

Said cover for wearing the excretion helping tool 200 is a diaper for wearing an insert-type toilet bowl for the same purpose as that in the present invention. Said excretion helping tool 100 is an insert-type toilet bowl for the same purpose as that in the present invention.

### Literatures of prior art

### Patent literatures

Patent literature 1: JP2008-142243A
Patent literature 2: JP2006-141590A
Patent literature 3: JP5254498B1

### General description of the invention

### Problem to be solved by the invention

Prior covers for wearing excretion helping tools 200 have following problems:
(1) In an excretion helping tool 100, an opening portion 140 comprising a horizontal defecated fecal receiving portion and a vertical discharged urine receiving portion is formed. The opening portion 140 and an opening portion 210 of the cover for wearing excretion helping tool 200 must be brought in line and the cover for wearing excretion helping tool must be attached to the inside of the excretion helping tool 100. However, because there is no standard for position adjustment between the excretion helping tool 100 and the cover for wearing excretion helping tool 200, the adjustment work to bring in line cannot be done easily and it takes time until the cared person wears the device.
(2) In case that the opening portion 140 of the excretion helping tool 100 and the opening portion 210 of the cover for wearing excretion helping tool 200 aren't brought in line, defecated feces attaches around the opening portion 210 or discharged urine scatters to wet around the opening portion 210, which causes to damage the wearing sensation and yield odor.
(3) When the cover for wearing excretion helping tool 200 is worn, especially in case of male, if the male sexual organ doesn't face the urination portion, the surrounding of the opening portion 210 may get wet.
(4) Although a leakage preventive barrier 270 and a three-dimensional gather 260 are formed around the opening portion 210, particularly, in persons who change the body position, urinary leakage occurs around the opening portion 210, by which bedclothes may get wet.
(5) In the cover for wearing excretion helping tool 200, a retentive sheet 250 attaches around the opening portion 210 to prevent that the periphery of the opening portion 210 of the cover 200 attaches firmly to the crotch (region between the legs). However, an anterior cup portion 120 is mounted at the excretion helping tool 100, so that regardless of body sizes such as fat or lean, the height is fixed because of the anterior cup portion 120. In persons with portly form, the upper region in the crotch may attach firmly to the cover for wearing excretion helping tool 200, which causes to get wet by urine or rinse solution and not to dry easily, leading to unpleasant sensation in the person. In such condition, its wearing for a long time is a problem.

The present invention intents to provide the diaper for inert-type toilet bowl, wherein the insert-type toilet bowl and the diaper for insert-type toilet bowl can be set up in a short time efficiently and the periphery of the defecated fecal receiving portion and discharged urine receiving portion don't get wet to maintain comfortable condition in wearing the diaper for a long time.

### Means for solving problem

The present invention is characterized by that an excretion hole 74 is formed at a main body of diaper 67 as corresponding to a defecated fecal receiving portion 14 in an insert-type toilet bowl 9 equipping the defecated fecal receiving portion 14 and a hip receiving portion 13; in a diaper for insert-type toilet 66 set at the inner side of said insert-type toilet bowl 9 for usage, a toilet-seat receiving sheet 78 for forming a plug-in hole 79 of said hip receiving portion 13 is laid on at the outer surface of said main body of diaper 67; and the depth of said plug-in hole 79 is formed in such a manner that said defecated fecal receiving portion 14 and the excretion hole 74 are brought in line when said hip receiving portion 13 is inserted; and also characterized by that said toilet seat receiving sheet 78 is made to have hygroscopic and waterproof properties and dimensions enabling wrapping at least the defecated fecal receiving portion 14 from the downside of the insert-type toilet bowl 9.

The invention is characterized by that the excretion hole 74 is formed at the nearly center of a crotch contour wrapping portion 70 in the diaper 66 for insert-type toilet bowl comprising a waist contour wrapping portion 68, a crotch contour wrapping portion 70, an abdominal contour wrapping portion 69 in the main body of the diaper 67; a male sexual organ retentive portion 80 is laid on as facing the excretion hole 74; and said male sexual organ retentive portion 80 is formed in such a manner that two parallel elongated protrusion bodies having mutual interspaces are directed toward the excretion hole 74.

The invention is characterized by that the excretion hole 74 is formed at the crotch contour wrapping portion 70 of the main body of the diaper 67 as corresponding to the defecated fecal receiving portion 14 and the discharged urine receiving portion 15 in the insert-type toilet bowl 9 equipping the defecated fecal receiving portion 14 and the discharged urine receiving portion 15; in the diaper for the insert-type toilet bowl 66 set inside said inset-type toilet bowl 9, a part of said crotch contour wrapping portion 70 is pulled and fixed at the side of the insert-type toilet bowl 9 in the outer surface of the crotch contour wrapping portion 70, facing said excretion hole 74 and a gap retentive tape 81 is laid on for forming a gap inside the crotch contour wrapping portion 70; and a gap retentive tape 82 to hold the gap retentive tape 81 is laid on in a direction to intersect with the gap retentive tape 81 in the outer surface of the crotch contour wrapping portion 70.

The invention is characterized by that the excretion hole 74 is formed at the nearly center of said crotch contour wrapping portion 70 in the diaper for insert-type toilet bowl 66 comprising the waist contour wrapping portion 68, the crotch contour wrapping portion 70 and the abdominal contour wrapping portion 69 in the main body of the diaper 67; an inner gather tape 77a and an outer gather tape 77b extending in the longitudinal direction of the main body of the diaper 67 as facing both the sides of said excretion hole 74 are laid on in the inner surface of said crotch contour wrapping portion 70; wherein the inner gather tape 77a and the outer gather tape 77b are configured in such a manner that the inner marginal portion and the bilateral marginal portions are attached to the main body of the diaper 67, respectively and the outer marginal portion is made from elastic materials and thus becomes contracting.

### Effect of the invention

According to the invention described in Claim 1, the excretion hole is formed at the main body of the diaper as corresponding to said defecated fecal receiving portion in the insert-type toilet bowl equipping the defecated fecal receiving portion and the hip receiving portion, the toilet seat receiving sheet for forming the plug-in hole of the hip receiving portion is laid on at the outer surface of the main body of the diaper in the diaper for insert-type toilet bowl used by setting inside said insert-type toilet bowl; and the depth of said plug-in hole is formed in such a manner that said defecated fecal receiving portion and the excretion hole are brought in line when said hip receiving portion is inserted. As the results, the defecated fecal receiving portion and the excretion hole of the diaper for insert-type toilet bowl can be brought in line only by inserting the hip receiving portion of the insert-type toilet bowl until it comes in contact with the back of the plug-in hole at the outer surface of the diaper for insert-type toilet bowl, and the adjustment work for bringing in line can be done very easily and the device can be worn smoothly and accurately in cared persons.

According to the invention described in Claim 2, the toilet seat receiving sheet has hygroscopic and waterproof properties and has dimensions enabling to wrap up at least the defecated fecal receiving portion from the downside of the insert-type toilet bowl. As the results, even caring persons who are unexperienced with devices for cared persons can operate for preventing the leakage easily and surely. The leakage doesn't occur even in change in body position.

According to the invention described in Claim 3, the excretion hole is formed at the crotch contour wrapping portion of the main body of the diaper as corresponding to said defecated fecal receiving portion and the discharged urine receiving portion in the insert-type toilet bowl equipping the defecated fecal receiving portion and the discharged urine receiving portion and a part of said crotch contour wrapping portion is pulled to the side of the insert-type toilet bowl and fixed on the outer surface of the crotch contour wrapping portion facing said excretion hole in the diaper for insert-type toilet bowl which is used by setting inside said insert-type toilet bowl and the gap retentive tape is laid on to form a gap inside the crotch contour wrapping portion. As the results, An appropriate gap can be formed according to body sizes regardless of fat or lean condition. The gap enables to prevent the attachment of defecated feces to the region around the excretion hole and wetting the periphery of the excretion hole by scattering of discharged urine and also prevent to damage the wearing sensation for a long time and the occurrence of odor.

According to the invention described in Claim 5, in the diaper for the insert-type toilet bowl comprising the waist contour wrapping portion, the crotch contour wrapping portion, and the abdominal contour wrapping portion in the main body of the diaper, the excretion hole is formed at the nearly center of said crotch contour wrapping portion and the inner gather tape and the outer gather tape extending toward the longitudinal direction of the main body of the diaper, respectively as facing both the sides of said excretion hole. As the results, discharged urine doesn't leak from the crotch contour wrapping portion externally and the occurrence of odor can be suppressed.

According to the invention described in Claim 6, the inner gather tape and the outer gather tape attach to the main body of the diaper by the inner marginal portion and the bilateral marginal portions, and the outer marginal portion is made from elastic materials to become contracting. As the results, the inner gather tape and the outer gather tape upstand at the time of the contraction by the elastic materials of the outer marginal portion and attach firmly to the femoral root, which ensures to prevent the leakage outside.

### Brief description of drawings

[Fig.1] This is a diagrammatic perspective view, showing a case of the embodiment of the diaper for insert-type toilet bowl 66 by the present invention.
[Fig.2] This is a diagrammatic perspective view seen from the outer side of the diaper for the insert-type toilet bowl 66 by the invention.
[Fig.3] This is the assembly working process of the diaper for insert-type toilet bowl 66 by the invention, in which (a-1) is a diagram seen from the inner side of the first process, (a-2) is an A-A line end elevational view, (b-1) is a diagram seen from the inner side of the second process, and (b-2) is a B-B line end elevational view.
[Fig.4] In the assembly working processes of the diaper for insert-type toilet bowl 66 by the invention, (c-1) is the diagram seen from the inner side of the third process, (c-2) is a C-C line end elevational view, and (d) is a diagram seen from the outer side of the fourth process.
[Fig.5] In the assembly working processes of the diaper for insert-type toilet bowl 66 by the invention, (e) is a diagram seen from the fifth process, and (f) is a diagram seen from the outer side of the sixth process.
[Fig.6] This is a diagrammatic perspective view of the insert-type toilet bowl 9 used with the diaper for insert-type toilet bowl 66 by the invention.
[Fig.7] This is a diagrammatic perspective view of the insert-type toilet bowl 9 with a bed-sore preventive member mounted, as shown in Fig.4.
[Fig.8] This is a cross section view of a condition that the diaper for insert-type toilet bowl 66 by the invention is set up at the insert-type toilet bowl 9.
[Fig.9] This is a disassembled diagrammatic perspective view of a prior excretion helping tool 100 and a cover for wearing excretion helping tool 200.

### Mode for carrying out the invention

In the diaper for insert-type toilet bowl 66 by the invention, the excretion hole 74 is formed at the main body of the diaper 67 as corresponding to said defecated fecal receiving portion 14 in the insert-type toilet bowl 9 equipping the defecated receiving portion 14 and the hip receiving portion 13, and in the diaper for insert-type toilet bowl 66 used by setting inside the insert-type toilet bowl 9, the toilet seat receiving sheet 78 for forming the plug-in hole 79 of the hip receiving portion 13 is laid on at the outer surface of said main body of the diaper 67. The depth of said plug-in hole 79 is formed in such a manner that said defecated fecal receiving portion 14 and the excretion hole 74 are brought in line when said hip receiving portion 13 is inserted. Said toilet seat receiving sheet 78 has hygroscopic and waterproof properties and dimensions by which at least the defecated fecal receiving portion 14 can be wrapped up from the downside of the insert-type toilet bowl 9.

In the diaper for insert-type toilet bowl 66 by the invention, comprising the waist contour wrapping portion 68, the crotch contour wrapping portion 70 and the abdominal contour wrapping portion 69 in the main body of the diaper 67, the excretion hole 74 is formed at the nearly center of said crotch contour wrapping portion 70 and the male sexual organ retentive portion 80 is laid on as facing the excretion hole 74. The male sexual organ retentive portion 80 is configured in such a manner that two parallel elongated protrusion bodies are directed toward the excretion hole 74.

In the diaper for insert-type toilet bowl 66 by the invention, the excretion hole 74 is formed at the crotch contour wrapping portion 70 of the main body of the diaper 67 as corresponding to said defecated fecal receiving portion 14 and the discharged urine receiving portion 15 in the insert-type toilet bowl 9 equipping the defecated fecal receiving portion 14 and the discharged urine receiving portion 15. In the diaper for insert-type toilet bowl 66 used by setting inside the said insert-type toilet bowl 9, a part of said crotch contour wrapping portion 70 is pilled to the side of the insert-type toilet bowl 9 and fixed at the outer surface of the crotch contour wrapping portion 70 facing said the excretion hole 74 and the gap retentive tape 81 for forming a gap inside the crotch contour wrapping portion 70 is laid on. The gap retentive tape 82 for holding the gap retentive tape 81 is laid on at the outer surface of the crotch contour wrapping portion 70 in a direction to intersect with the gap retentive tape 81.

In the diaper for insert-type toilet bowl 66 by the invention, comprising the waist contour wrapping portion 68, the crotch contour wrapping portion 70, and the abdominal contour wrapping portion 69 in the main body of the diaper 67, the excretion hole 74 is formed at the nearly center of said crotch contour wrapping portion 70 and the inner gather tape 77a and the outer gather tape 77b extending to the longitudinal direction of the main body of the diaper 67 are laid on as facing the both the sides of said excretion hole 74. The inner gather tape 77a and the outer gather tape 77b attach to the main body of the diaper 67 by the inner marginal portion 70 and the bilateral marginal portions, respectively and the outer marginal portion 70 is made from elastic materials to become contracting.

### Embodiment case 1

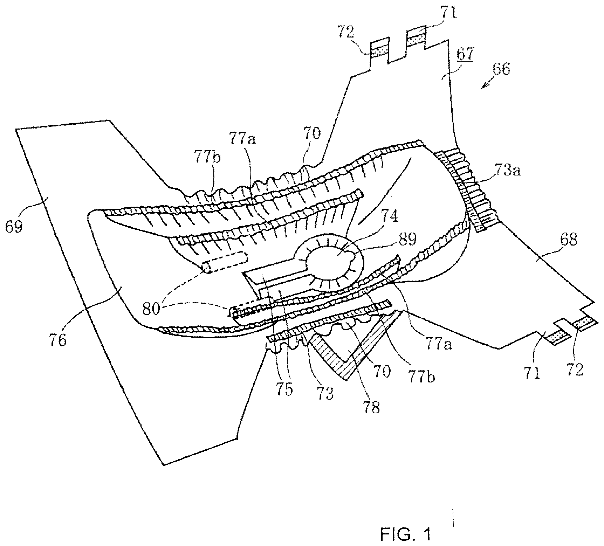

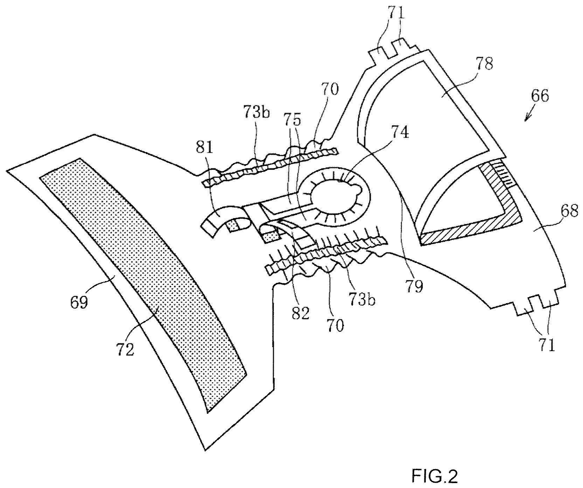

In Fig.1, (a-1) is a diagrammatic perspective view seen from the inner side after the diaper for insert-type toilet bowl 66 by the invention was assembled. Fig.2, (a-2) is also a diagrammatic perspective view seen from the outer side.

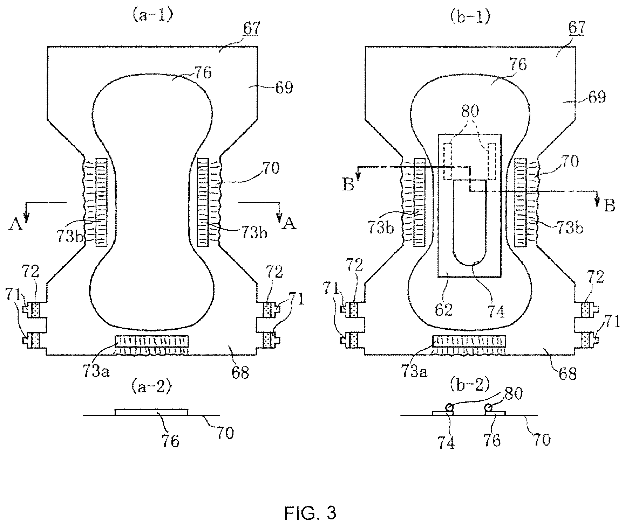

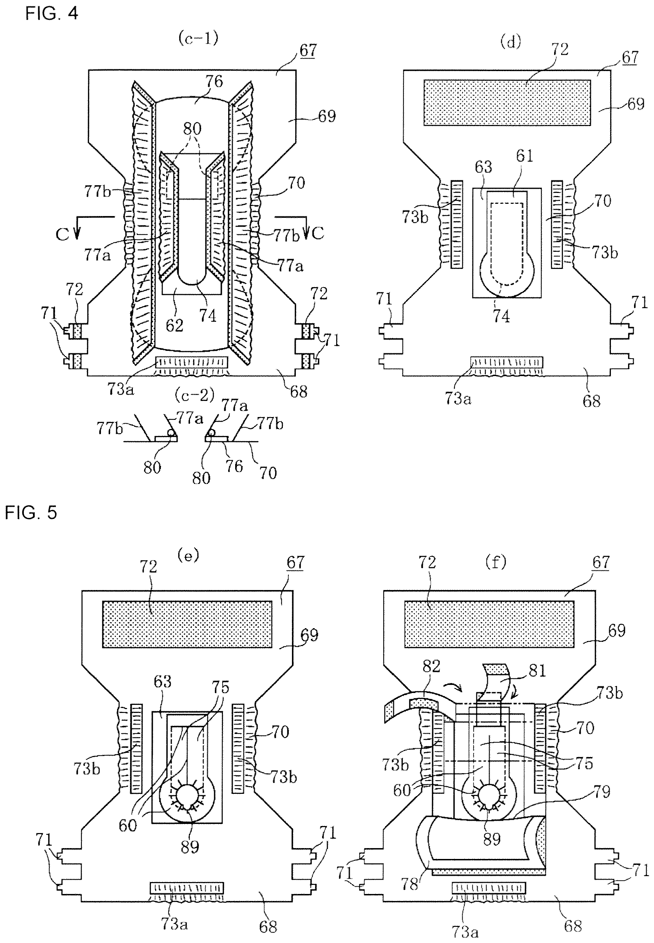

Based on Fig.3 and Fig.5, the procedures for assembling the diaper for insert-type toilet bowl 66 is explained in order of each process.

### (1) The first process

In Fig.3, the diagram seen from the inner ide of the diaper for insert-type toilet bowl 66 is an A-A line end elevational view.

The main body of the diaper 67 which is thin and strong and made of paper is prepared.

The main body of the diaper 67 which is 55∼60cm in width and 80∼85cm in length is trisected in a longitudinal direction into a waist contour wrapping portion 68 at the front side, an abdominal contour wrapping portion 69 at the back side and a crotch contour wrapping portion 70 at the middle position, in which the crotch contour wrapping portion 70 is 35∼40cm with constriction. A portion near the end of said waist contour wrapping portion 68 becomes a waist gather 73a of about 20cm in extension which is made from elastic materials such as rubber etc., and similarly, a portion at both the sides of the crotch contour wrapping portion 70 becomes a crotch gather 73b of about 20cm in extension which is made from elastic materials such as rubber etc., in which the length without extension, the constricted length, is about 12cm. Inside the main body of the diaper 67, a thick cushion member 76 as shown in (a-2) is attached to a region covering the waist contour wrapping portion 68, the crotch contour wrapping portion 70 and the waist contour wrapping portion 68. A fixing members 71 with two face fasteners each 72 are laid on at the bilateral marginal portions of the waist contour wrapping portion 68 in a projected manner.

### (2) The second process

In Fig.3, (b-1) is a diagram seen from the inner side of the diaper for insert-type toilet bowl 66 and (b-2) is a B-B line end elevational view.

A male sexual organ retentive portion 80 comprising elongated protrusion bodies made from soft materials rounded with about 10cm in length and about 2cm in diameter is attached to a portion on the cushion member 76 in the longitudinal direction with an interval of about 10cm in the border region between said crotch contour wrapping portion 70 and the abdominal contour wrapping portion 69. An inner waterproof sheet 62 of about 16cm in width and about 10cm in length is attached to a nearly central position in the crotch contour wrapping portion 70 as said male sexual organ retentive portion 80 is retained. The overall bell-shape excretion hole 74 in which the bottom end portion is a half-circle shape of about 8∼10cm (almost the same size as the diameter of the defecated fecal receiving portion 14 of the insert-type toilet bowl 9 described later) and the upper end portion is a rectangle of about 30cm penetrates the cushion member 76 and the crotch contour wrapping portion 70 in the setting.

### (3) The third process

In Fig.4, (c-1) is a diagram seen from the inner ide of the diaper for insert-type toilet bowl 66 and (c-2) is a C-C line end elevational view.

The inner gather tape 77a of about 5cm in width is attached along the line of the inner marginal portion of the excretion hole 74 at the upper surface of the inner waterproof sheet 62. In the bilateral inner gather tapes 77a, the inner marginal portion and both the end portions are attached, and the outer marginal portion which isn't attached constricts by elastic materials such as rubber etc., so that the marginal portion gets up at the side of the excretion hole 74, as shown in Fig.4(c-2). In regions outside the inner gather tapes 77a, the outer gather tapes 77b are attached to a long region from the waist contour wrapping portion 68 to the abdominal contour wrapping portion 69 through the crotch contour wrapping portion 70. In the outer gather tapes 77b, similarly to the inner gather tape 77a, the inner marginal portion and both end portions are attached and the outer marginal portion which is not attached constricts by elastic materials such as rubber etc., so that the outer marginal portion gets up at the inner side, as shown in Fig.4 (c-2).

### (4) The fourth process

In Fig.4, (d) is a diagram seen from the upside of the diaper for insert-type toilet bowl 66. An outer water waterproof sheet 63 of about 18cm in width and about 30cm in length is attached to the outer surface of the main body of the diaper 67 as covering the excretion hole 74. A bottle-gourd shape toilet-seat fixing sheet 61 is attached to a region on the outer waterproof sheet 63. An adhesive agent is coated on the outer surface of the toilet seat fixing sheet 61 which ic protected by peel-off sheet.

### (5) The fifth process

In Fig.5, (e) is a diagram seen from the outer side of the diaper for insert-type toilet bowl 66. A score 60 is made at the outer waterproof sheet 63 and the toilet seat fixing sheet 61. As for the score 60, a plunger helix 89 of slightly smaller circle than the half circles (for exposing the defecated fecal receiving portion 14 of the insert-type toilet bowl 9 described later) is laid on at the half-circle portion of the excretion hole 74, and a plunger helix 89 of a small half-circle shape (for exposing a rinse solution nozzle 17 of the insert-type toilet bowl 9 described later) is laid on at the bottom end of the circular portion. In the rectangular portion of the excretion hole 74, the T-letter shape score 60 is formed by longitudinal straight line at the center and transverse straight line at the upper margin, and moreover, a radial score 60 is formed around the plunger helix 89. Forming such scores 60 enables to make an adhesive member 75 which can be folded back around the excretion hole 74. A thin and long face fastener 72 of about 12 x 42cm is attached to the abdominal contour wrapping portion 69.

### (6) The sixth process

In Fig.5, (f) is a diagram seen from the outside of the diaper for insert-type toilet bowl 66.

In the waist contour wrapping portion 68, the hygroscopic and waterproof toilet seat receiving sheet 78 is attached to the peripheral portion of the three sides in the about inferior half region and an adhesive agent is coated at the peripheral portion of the three sides in the about superior half region, which are protected by peel-off sheet. At that time, the plug-in hole 79 is formed between the waist contour wrapping portion 68 and the toilet seat receiving sheet 78. The width of the toilet seat receiving sheet 78 is a size with which the hip receiving portion 13 of the insert-type toilet bowl 9 described later can be inserted from the plug-in hole 79 smoothly. The depth of the plug-in hole 79 is made a distance with which when the hip receiving portion 13 is inserted to the back of the plug-in hole 79, the plunger helix 89 of the diaper for insert-type toilet bowl 66 becomes congruent with the defecated fecal receiving portion 14 of the insert-type toilet bowl 9. The length of the upper half of the toilet seat receiving sheet 78 is prolonged to be able to cover at least the defecated fecal receiving portion 14 of the insert-type toilet bowl 9 and a port of the discharged urine receiving portion 15.

In the upper portion of the toilet seat fixing sheet 61, a vertical gap retentive tape 81 adheres to the upper end, and the inside of the inferior end portion is coated by adhesive agent, which is protected by peel-off sheet. In the slightly inferior side of the gap retentive tape 81, a transverse gap retentive tape 82 adheres to one end in such a manner to intersect with the gap retentive tape 81, the nearly central portion and the inside of the other end portion are coated by adhesive agent, which are protected by peel-off sheet.

Next, an example of the insert-type toilet bowl 9 used with diaper for insert-type toilet bowl 66 in the present invention is explained.

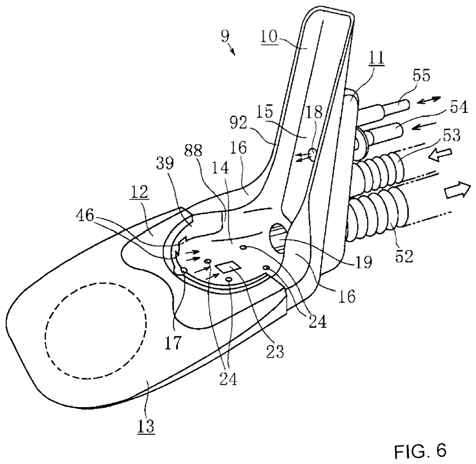

In Fig.6, the insert-type toilet seat 9 comprises a main body of toilet seat 10, a back cover portion 11, an anterior cover portion 12, and the hip receiving portion 13 in broad terms.

The main body of the toilet seat 10 comprises the defecated fecal receiving portion 14 having a pateliform hollow of about 9∼10cm in diameter and the longitudinal canaliform discharged urine receiving portion 15 of about 5cm in width, about 17cm in height, and about 2cm in depth which is at the right angle to the defecated fecal receiving potion 14 or tilts slightly backward to the right angle.

Said defecated fecal receiving portion 14 tilts slightly toward the back end from the front end, communicates with the back end, and projects in one with a tubular portion becoming the defecated fecal suction portion 19. A rinse solution nozzle 17 is laid on at the front end of the defecated fecal receiving portion 14. A wide femoral receiving portion 16 of about 2∼3cm is laid on in a region from the bilateral marginal portions of said defecated fecal receiving portion 14 to the marginal portion of said discharged urine receiving portion 15 as tilting slightly toward both the sides. A circuit board which isn't shown in a diagram is set in the back surface of the defecated fecal receiving portion 14. A fecal sensor 23 mounted in the circuit board is exposed to the nearly center of the upper surface of the defecated fecal receiving portion 14 and four urine sensors 24 are exposed to a portion around the fecal sensor 23.

An upward spray nozzle cleansing the anus and pubes is mounted at an angle of 40∼50 degree to the horizon in the upper end portion of the rinse solution nozzle 17. In the side portion, a horizontal spray nozzle of a wide angle, about 180 degree, is mounted as spraying over the whole upper surface of the defecated fecal receiving portion 14. The bottom end portion of the rinse solution nozzle 17 is connected to a water pipe connected to a water pipe 54 described later.

A rinse solution nozzle 18 cleansing the anus and pubes is mounted at the nearly middle position of the discharged urine receiving portion 15. A low-friction material such as Teflon (registered brand name) etc. is coated on the surface of the defecated fecal receiving portion 14. The coating enables to prevent the attachment of feces and urine and facilitate the flow.

Said back cover portion 11 is fixed in a condition of slight gaps with the bottom side, back side and said wall of the toilet seat main body 10 and is mounted from the back side of the defected fecal receiving portion 14 and the discharged urine receiving portion 15 in said toilet seat main body 10. In the back side portion of the back cover portion 11, an excretory substance suction hose 52, an air supply pipe 53, and the water pipe 54, and an electric cord 55 are arranged in order from the downside.

Said excretory substance suction hose 52 is made from flexuous materials, which facilitates it that the cared person bends over and stands up as remaining in wearing the insert-type toilet bowl 9.

In said air supply pipe 53, two air supply pipes passing through the main body of the toilet seat 10 and the inside of the back cover portion 11 are mounted and are connected to a air blasting route 46 in the defecated fecal receiving portion 14. The warm water pipe 54 from said outside portion communicates with the rinse solution nozzles 17 and 18. The rinse solution nozzle 18 is exposed to the nearly center of the discharged urine receiving portion 15 and cleanses the male or female sexual organ after urination. Said electric cord 55 is connected to the fecal sensor 23 and the urine sensor 24 and conducts input and output of electric signals and supply of electric power etc.

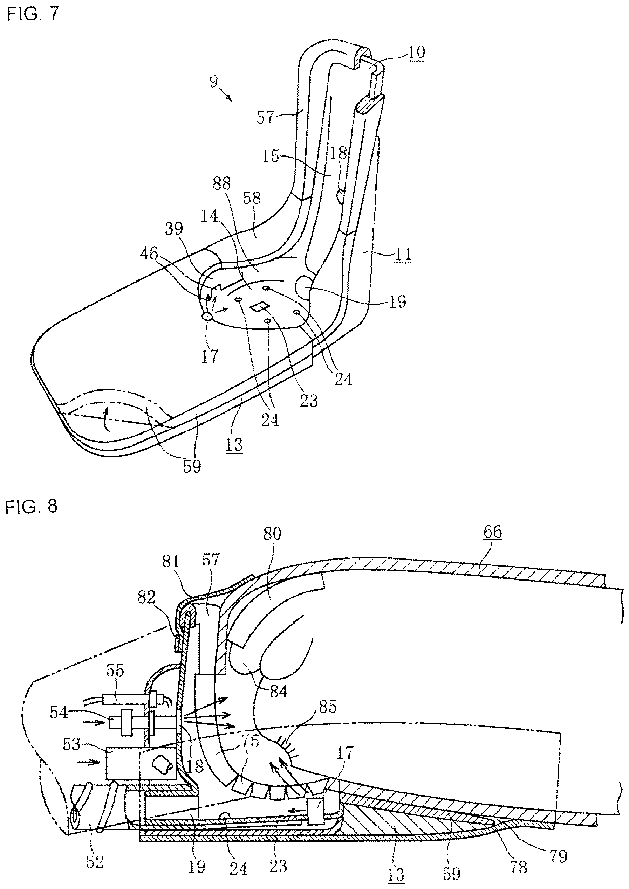

Next, as shown in Fig.7, a first bed-sore preventive member 57 for preventing bed sore, a second bed-sore preventive member 58, and a third bed-sore preventive member 59 are fitted in portions where the skins are compressed when the insert-type toilet bowl is worn. The first bed-sore preventive member 57 is covered at the bilateral marginal portions of the discharged urine receiving portion 15 from the upside, and the second bed-sore preventive member 58 is covered at the upper portion of the femoral receiving portion 16 from the upside. The first and second bed-sire preventive members 57 and 58 which are formed together in one is also usable.

When the first, second and third bed-sore preventive members 57, 58, and 59 are worn with the diaper for inert-type toilet bowl 66, it is desirable that the body pressure becomes 50mmHg or less, more preferably 32mmHg or less.

Procedures for setting up the insert-type toilet bowl 9 and the diaper for insert-type toilet bowl 66 which are configured as described above are explained below.
(1) The hip receiving portion 13 of the insert-type toilet bowl 9 shown in Fig.7 is inserted to the plug-in hole 79 of the diaper for insert-type toilet bowl 66 shown in Fig.8 until the tip bumps into the back surface. And the excretion hole 74 of the diaper for insert-type toilet bowl 66 fits in the defecated fecal receiving portion 14 accurately and the rinse solution nozzle 17 is exposed from the plunger helix 89 of the excretion ole 74. In this condition, the peel-off sheet of an adhesive member 75 in the periphery of the circular hole of the excretion hole 74 is peeled off and is then adhered to the cylinder cowling portion 39 of the anterior cover portion 12. The peel-off sheet of the adhesive member 75 at the straight portion of the excretion hole 74 is peeled off and is then adhered to the inner wall portions 88 of the defecated fecal receiving portion 14 and the discharged urine receiving portion 15.
(2) The diaper for insert-type toilet bowl 66 and the insert-type toilet bowl 9 are set up and it is applied to the crotch portion between the legs in the cared person. At that time, when the gluteal portion is placed on the third bed sore preventive member 59 of the hip receiving portion 13, the anus 85 becomes almost directly above the fecal sensor 23 of the defecated fecal receiving portion 14 and the femoral root portion is attached firmly to the second bed-sore preventive member 58 covered at the femoral receiving portion 16 in the insert-type toilet bowl 9 through the diaper for the insert-type toilet bowl 66. Two pieces each of inner gather tapes 77a and outer gather tapes 77b upstand at both the sides of the excretion hole 74 and are attached firmly to the femoral root portion to prevent leakage of urine or rinse solution or leakage of odor as far as possible.
(3) Once the firm attachment is made, the abdominal contour wrapping portion 69 is folded back up to the upside of the abdomen in the cared patient. At that time, in case of male patient etc., the male sexual portion is placed at the portion in the male sexual organ retentive portion 80 and the urinary tract is placed correctly toward the discharged urine receiving portion 15. In case of female patient etc., the female sexual organ is faced off just about against the rinse solution nozzle 18 of the discharged urine receiving portion 15.
(4) The abdominal contour wrapping portion 69 is winded up from the upside of the abdomen in the cared patient etc., and the waist contour wrapping portion 68 is covered as a waist gather 73a is extended from both the sides of the abdominal contour wrapping portion 69, and a face fastener 72 of the fixing member 71 is latched together with a face fastener 72 of the abdominal contour wrapping portion 69.
(5) To prevent the attachment of feces or urine to the diaper for insert-type toilet bowl 66 around the excretion hole 74 by forming appropriate spaces among the defecated fecal receiving portion 14 and the discharged urine receiving portion 15 in the insert-type toilet bowl 9 and the urinary organs in the cared patient etc., the gap retentive tape 81 is pulled, and as the outer surface of the diaper for insert-type toilet bowl is attached firmly to the first bed-sore preventive member 57 on the insert-type toilet bowl 9, the peel-off sheet of the gap retentive tape 81 is removed and is then adhered to the back surface of the toilet set main body 10, and moreover, is intersected with the gap retentive tape 81 and the gap retentive tape 82 is adhered to the outer surface of the toilet seat main body 10 in order to avoid for the gap retentive tape 81 to become unstick. At that time, as shown in Fig.7, although the upper end portion of the discharged urine receiving portion 15 tilts slightly backward, because the frontal surface of the first bed sore preventive member 57 is nearly vertical, an appropriate space is formed in a portion between the discharged urine receiving portion 15 and the urinary organs of the cared patient etc.
   In the diaper for insert-type toilet bowl 66, two pieces each of the inner gather tape 77a and the outer gather tape 77b are formed at both the sides of the excretion hole 74 and crotch portion gather 73b are formed at both the sides of the crotch contour wrapping portion 70. As the results, Feces, urine or rinse solution hardly leaks from the diaper for insert-type toilet bowl 66.
(6) However, moreover, to prevent to leak from the defecated fecal receiving portion 14 and the discharged urine receiving portion 15 because of insufficient setting of the diaper for insert-type toilet bowl 66 and the plug-in toilet bowl 9, the toilet seat receiving sheet 78 of the outer surface of the diaper for insert-type toilet bowl 66 is prolonged to wrap up the outer surface of the defecated fecal receiving portion 14 and the peel-off sheet of the toilet seat receiving sheet 78 is removed and is attached to the toilet seat main body 10 and the back cover portion 11 without any gap. The toilet seat receiving sheet 78 which has waterproof and water absorption functions enables to surely prevent the leakage to the outside the plug-in toilet bowl 9.

As described above, when the diaper for insert-type toilet bowl 66 and the plug-in toilet bowl 9 are set up and the fecal sensor 23 or the urine sensor 24 detects defecated feces or discharged urine, an excretory substance processing device which is not shown in a diagram drives and the feces and urine inside the insert-type toilet bowl 9 is sucked and processed in the defecated fecal suction hole 19 and cleansing by warm water and drying by warm wind are conducted.

### Explanations of letters and numerals

10···Toilet seat main body, 11···Pack cover portion, 12···Anterior cover portion, 14··· Defecated fecal receiving portion, 15···Urine receiving portion, 16···Femoral receiving portion, 17,18···Rinse solution nozzle, 19···Fecal suction hole, 23···Fecal sensor, 24··· Urine sensor, 39··· Cylinder cowling portion, 46···Air blasting route, 52···Excretory substance suction hose, 53···Air supply hose, 54···Water pipe, 55···electric cord, 57···First bed-sore preventive member, 58··· Second bed-sore preventive member, 59···Third bed-sore preventive member, 60···Score, 61··· Toilet seat adhesive sheet, 62···Inner waterproof sheet, 63···Outer waterproof sheet, 66···Diaper for insert-type toilet bowl, 67···Diaper main body, 68···Waist contour wrapping portion, 69··· Abdominal contour wrapping portion, 70···Crotch contour wrapping portion, 71···Fixing member, 72···Face fastener, 74···Excretion hole, 75···Adhesive member, 76···Cushion member, 78···Toilet seat receiving sheet, 79···Plug-in hole, 80···Maie sexual organ retentive portion, 81···Gap retentive tape, 82···Gap retentive portion, 84···Male sexual organ, 85···Anus, 88···Inner wall portion, 89···Plunger helix

## Claims

1. A diaper for insert-type toilet bowl comprising a defecated fecal receiving portion and a hip receiving portion, wherein an excretion hole is formed at the diaper main body corresponding to said defecated fecal receiving portion, a toilet seat receiving sheet is laid on to form a plug-in hole of said hip receiving portion at the outer surface of said diaper main body in the diaper for insert-type toilet bowl used set at the inner side of said insert-type toilet bowel, and the depth of said plug-in hole is configured in such a manner that the said hip receiving portion is inserted, said defecated fecal receiving portion and the excretion hole get together.

2. The diaper for insert-type toilet bowl according to the description in Claim 1, wherein the toilet seat receiving sheet has hygroscopic and waterproof properties and has dimensions by which al least the defecated fecal receiving portion can be wrapped up from the downside of the insert-type toilet bowl.

3. The diaper for insert-type toilet bowl comprising the defecated fecal receiving portion and a discharged urine receiving portion, wherein the excretion hole is lain on at a crotch contour wrapping portion of the diaper main body corresponding to said defecated fecal receiving portion and the discharged urine receiving portion; in the diaper for insert-type toilet bowl used set at the inner side of said insert-type toilet bowl, a part of said crotch contour wrapping portion is pulled to the side of the insert-type toilet bowel and fixed on the outer surface of the crotch contour wrapping portion facing said excretion hole and a gap retentive tape for configuring gaps inside the crotch contour wrapping portion is laid on.

4. The diaper for insert-type toilet bowl according to the description in Claim 3, wherein a gap retentive tape for retaining the gap retentive tape is laid on at the outer surface of the crotch contour wrapping portion in a direction to intersect with the gap retentive tape.

5. The diaper for insert-type toilet bowl comprising a waist contour wrapping portion, the crotch contour wrapping portion and an abdominal contour wrapping portion in the diaper main body portion, wherein the excretion hole is formed at the nearly center of said crotch contour wrapping portion, and an inner gather tape and an outer gather tape extending to the longitudinal direction of the diaper main body, respectively, as facing both the sides of said excretion hole.

6. The diaper for insert-type toilet bowl according to the description in Claim 5, wherein the inner gather tape and the outer gather tape adhere to the diaper main body by the inner marginal portion and bilateral marginal portions and the outer marginal portion is made from elastic materials to become constricting.
